# EUROPÄISCHE PATENTSCHRIFT

(11) **EP 1 036 557 B1**
(45) Veröffentlichungstag und Bekanntmachung des Hinweises auf die Patenterteilung: **11.04.2001**
(21) Anmeldenummer: 00102661.6
(22) Anmeldetag: 08.02.2000
(51) Int. Cl.: A61K 7/13

(54) **Voremulsion und Verwendung derselben zur Herstellung eines Haarfärbemittels sowie Verfahren zur Herstellung einer Haarfärbeemulsion**
Preemulsion and its use for making hair dyes as well as a method for making hair dye emulsions
Préémulsion et son utilisation pour fabriquer une teinture de cheveux ainsi qu'une méthode de fabrication d'une émulsion pour teindre les cheveux

(30) Priorität: 11.02.1999 DE 19905768
(43) Veröffentlichungstag der Anmeldung: 20.09.2000
(73) Patentinhaber: GOLDWELL GmbH, 64297 Darmstadt (DE)
(72) Erfinder: Lorenz, Heribert, 64401 Gross-Bieberau (DE)

(56) Entgegenhaltungen:
- DE-A- 3 834 142
- DE-A- 4 103 292
- DE-A- 4 103 551
- DE-A- 19 511 571
- GB-A- 2 129 447
- US-A- 5 525 123

## Beschreibung

Die vorliegende Erfindung betrifft eine Voremulsion und ein Verfahren zur Herstellung eines Haarfärbemittels auf Basis einer feinen wäßrigen Emulsion, enthaltend mindestens ein Oxidationsfarbstoff-Vorprodukt.

Permanente Haarfärbemittel auf Basis von Oxidationsfarbstoffen erfreuen sich einer weiten Verbreitung. Ihre Anwendung erfolgt in der Regel dergestalt, daß eine flüssige, zumeist als wäßrige Emulsion vorliegende, mindestens ein Oxidationsfarbstoff-Vorprodukt, im allgemeinen mindestens eine Entwickler- und mindestens eine Kupplersubstanz enthaltende Zusammensetzung unmittelbar vor der Anwendung mit einer Peroxid enthaltenden Zusammensetzung vermischt und die Mischung auf das Haar aufgebracht wird.

Dabei ist es wünschenswert, diese Emulsionen auf möglichst einfache Weise herzustellen und möglichst einfach zusammengesetzte, gleichwohl stabile Produkte zu erhalten.

Die Erfindung geht daher von der Aufgabenstellung aus, ein einfaches Verfahren zur Herstellung von Haarfärbeemulsionen zur Verfügung zu stellen, das zeit- und energiesparend arbeitet, wobei die erhaltenen Emulsionen auch eine gute Vermischbarkeit mit der Oxidationsmittel-Zusammensetzung, d.h. im Regelfall wäßrigem Wasserstoffperoxid, gewährleisten, was wiederum zu einer gleichmäßigen Verteilung des fertigen Färbemittels auf dem Haar und damit zu einer guten Färbeleistung führt.

Die erfindungsgemäße Lösung dieser Aufgabe besteht in der Anwendung eines Verfahrens zur Herstellung eines emulsionsförmigen Haarfärbemittels, wobei bei einer Temperatur von maximal 60°C, insbesondere etwa 30 bis etwa 50°C, eine Voremulsion aus bestimmten Emulgatoren und Fetten mit einer wäßrigen Zusammensetzung, die mindestens ein Oxidationsfarbstoff-Vorprodukt enthält, unter Rühren vermischt wird.

Das bevorzugte Gewichtsverhältnis zwischen Voremulsion und wäßriger Zusammensetzung liegt bei etwa 15 bis 40 und 85 zu 60, insbesondere etwa 1 zu 2.

Diese Voremulsion enthält
a) 20 bis 40 Gew.-% mindestens eines C₁₂-C₁₄-Fettalkoholethoxylats mit 1 bis 5 Ethylenoxidgruppen;
b) 15 bis 40 Gew.-% Ölsäure;
c) 5 bis 30 Gew.-% Ethandiol- und/oder 1,2-Propandioldistearat;
d) 5 bis 20 Gew.-% Glycerylstearat und/oder mindestens einen Zuckerfettsäureester; und
e) 0 bis 25 Gew.-% mindestens eines C₁₂-C₁₈-Fettalkohols,
   jeweils berechnet auf die Gesamtzusammensetzung der Voremulsion; keine weiteren Emulgatoren oder Fettkörper, und weist einen Wassergehalt von maximal 10 Gew.-%, insbesondere maximal 5 Gew.-%, auf, wobei der pH-Wert vorzugsweise durch Ammoniak und/oder Ethanolamin(e), auf 7,1 bis 9 eingestellt ist.

Als Bestandteil a) werden vorzugsweise etwa 25 bis 35 Gew.-% C₁₂-C₁₄-Fettalkoholethoxylat, insbesondere Laureth-2 oder Myristeth-3, eingesetzt.

Die Ölsäure ist in der erfindungsgemäßen Voremulsion vorzugsweise ebenfalls in einer Menge von etwa 25 bis 35 Gew.-% enthalten.

Als Bestandteil c) wird vorzugsweise Ethandioldistearat, insbesondere in einer Menge von 10 bis 25 Gew.-%, eingesetzt.
Als Bestandteil d) werden insbesondere 5 bis 15 Gew.-% Glycerylstearat, Saccharose- und Glucoseester wie Glucose- und Methylglucosedioleat, Methylglucosesesquistearat und/oder Polyglycoryl-3 -methylglucosedistearat eingesetzt.
Soweit als Bestandteil e) ein C₁₂-C₁₈-Fettalkohol vorhanden ist, handelt es sich vorzugsweise um Lauryl-, Myristyl-, Cetyl- und Cocosfettalkohol, vorzugsweise in einer Menge von 5 bis 20 Gew.-%.

Durch die Verwendung dieser einfach zusammengesetzten Voremulsion zur Herstellung von Haarfärbemitteln ist es möglich, alle benötigten Farbtöne durch Vermischen derselben mit den wäßrigen Haarfärbelösungen energiesparend bei niedriger Temperatur herzustellen.

Die Herstellung der Voremulsion erfolgt durch Zusammenrühren der geschmolzenen Bestandteile mit einem geringen Anteil an Wasser, d.h. bis zu maximal 10, vorzugsweise nicht mehr als 5, insbesondere maximal etwa 3 Gew.-%, das beispielsweise als 25%-ige Ammoniaklösung zur. Einstellung des pH-Wertes auf 7,1 bis 9, insbesondere etwa 7,5 bis 8,5, zugesetzt werden kann.
Die pH-Wert-Einstellung kann auch durch ein Ethanolamin, insbesondere Monoethanolamin, erfolgen.
Die Viskosität der erfindungsgemäßen Voremulsionen liegt vorzugsweise bei etwa 5.000 und 30.000, insbesondere bei etwa 10.000 bis 25.000, beispielsweise bei etwa 15.000 bis 20.000, mPa.s, gemessen bei 20°C im Brookfield-Viskosimeter BVT.

Die Wasserphase der einsatzfähigen, Oxidationsfarbstoff-Vorprodukte enthaltenden Endprodukts kann wasserlösliche Emulgatoren enthalten.
Als solche können insbesondere anionische Tenside verwendet werden.
Geeignete anionaktive Tenside sind insbesondere in einer Menge von etwa 0,25 bis etwa 5 Gew.-%, vorzugsweise 0,4 bis 2,5 Gew.-% der Gesamtzusammensetzung (einsatzfertige Emulsion), enthalten.
Dabei handelt es sich um solche vom Sulfat-, Sulfonat-, Carboxylat- und Alkylphosphat-Typ, vor allem natürlich diejenigen, die in Haarbehandlungsmitteln üblicherweise zum Einsatz gelangen, insbesondere die bekannten C₁₀-C₁₈-Alkylsulfate und die entsprechenden Ethersulfate, beispielsweise C₁₂-C₁₄-Alkylethersulfate, Laurylethersulfate, insbesondere mit 1 bis 4 Ethylenoxidgruppen im Molekül, Acylaminocarbonsäuren wie Lauroylsarkosinat und -glutamat, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.

Im Rahmen der Erfindung weiterhin geeignete anionische Tenside sind α-Olefinsulfonate bzw. deren Salze und Alkalisalze von Sulfobernsteinsäurehalbestern, beispielsweise das Dinatriumsalz des Monooctylsulfosuccinats, und Alkalisalze langkettiger Monoalkylethoxysulfosuccinate.

Geeignete Tenside vom Carboxylat-Typ sind Alkylpolyethercarbonsäuren und deren Salze der Formel

R-(C₂H₄O)ₙ-O-CH₂-COOX,

worin R eine C₈-C₂₀-Alkylgruppe, vorzugsweise C₁₂-C₁₄-Alkylgruppe, n eine Zahl von 1 bis 20, vorzugsweise 2 bis 17, und XH oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren.
Derartige Produkte sind seit längerem bekannt und im Handel, beispielsweise unter den Handelsnamen "AKYPO®" und "AKYPO-SOFT®".
Auch C₈-C₂₀-Acylisethionate können, allein oder im Gemisch mit anderen Tensiden, eingesetzt werden, ebenso Sulfofettsäuren und deren Ester.

Gegebenenfalls können auch amphotere bzw. zwitterionische Tenside als wasserlösliche Emulgatoren eingesetzt werden, insbesondere im Gemisch mit anionaktiven Tensiden, wobei die Gesamtmenge vorzugsweise bei etwa 0,25 bis 5, insbesondere etwa 0,5 bis 2,5 Gew.-% der Färbeemulsion liegen soll.

Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoactetat, Cocoaminopropionat und Natriumcocoamphopropinat und -acetat haben sich als geeignet erwiesen.

Auch die Verwendung nichtionischer wasserlöslicher Tenside, z.B. von C₈-C₁₈-Alkylpolyglucosiden mit einem Polymerisationsgrad von 1 bis 5, ist in den genannten Mengen alleine oder im Gemisch mit anionischen und/oder amphoteren bzw. zwitterionischen oberflächenaktiven Substanzen möglich.
Auch Aminoxide sind einsetzbar.
Weitere geeignete Tenside sind auch kationische Tenside wie die bekannten quaternären Ammoniumverbindungen mit einer oder zwei Alkyl- bzw. Alkenylgruppen mit 10 bis 22 Kohlenstoffatomen im Molekül, insbesondere in einer Menge von 0,1 bis 7,5, vorzugsweise 0,25 bis 5, besonders bevorzugt 0,5 bis 2,5 Gew.-%, der Gesamtzusammensetzung.

Die durch Verwendung der erfindungsgemäßen Voremulsion hergestellte Haarfärbeemulsion enthält mindestens ein Oxidationsfarbstoffvorprodukt, zweckmäßigerweise ein Gemisch aus mindestens einer Entwickler- und mindestens einer Kupplersubstanz.

Diese sind an sich bekannt und beispielsweise in der Monographie von K. Schrader, "Grundlagen und Rezepturen der Kosmetika", 2. Aufl. (1989), S. 784-799, beschrieben

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2'-Hydroxyethylamino)-5-aminotoluol und 1-Amino-4-bis-(2'-hydroxyethyl) -aminobenzol bzw. deren wasserlöslichen Salze; beispielhafte Kupplersubstanzen sind Resorcin, 2-Methylresorcin, 4-Chlorresorcin, 2-Amino-4-chlorphenol, 4-N-Methylaminophenol, 2-Aminophenol, 3-Aminophenol, 1-Methyl-2-hydroxy-4-amino-benzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diamindophenylamin, 2 -Dimethylamino-5-aminopyridin, 2,6-Diaminopyridin, 1,3-Diaminobenzol, 1-Amino-3-(2'hydroxyethyl-amino)benzol, 1-Amino-3-[bis(2'-hydroxyethyl)amino]benzol, 1,3-Diaminotoluol, α-Naph-thol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methlendioxybenzol, 2,4-Diamino-3-chlorphenol, und/oder 1-Methoxy-2-amino-4-(2'-hydroxyethylamino)-benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in der erfindungsgemäßen Farbemulsion kann je4weils etwa 0,1 bis etwa 5 Gew.-%, je nach gewünschter Färbung betragen.

Diese Oxidationsfarbstoffvorprodukte sind zweckmäßigerweise bereits in der wäßrigen Phase enthalten; sie können jedoch auch, falls erwünscht, der Fertigemulsion zugesetzt werden.

Die erfindungsgemäß hergestellten Färbezusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 insbesondere 0,1 bis 1 Gew.-% der Farbzusammensetzung (ohne Oxidationsmittel).

Die erfindungsgemäß hergestellten Haarfärbemittel können die in solchen Mitteln üblichen Grund- und Zusatzstoffe, Konditioniermittel, Stabilisatoren, Fette und Öle, Verdickungsmittel, Komplexbildner, etc. enthalten, die dem Fachmann aus dem Stand der Technik bekannt und beispielsweise in der Monographie von K. Schrader "Grundlagen und Rezepturen der Kosmetika", 2. Aufl. (Hüthig Buch Verlag, Heidelberg, 1989), S. 782 bis 815, beschrieben sind.

Die erfindungsgemäß hergestellten Färbeemulsionen weisen vorzugsweise einen im alkalischen Bereich liegenden pH-Wert, insbesondere zwischen etwa 8 und etwa 12,5, vorzugsweise zwischen 8,5 und 11, auf.

Zur Applikation wird die erfindungsgemäß hergestellte Oxidationsfarbstoff-Vorprodukt-Emulsion mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d.h. nach Vermischen mit Peroxid, kann sowohl im schwach sauren, d.h. einem Bereich von 5,5 bis 6,9, im neutralen als auch im alkalischen Bereich, d.h. zwischen pH 7,1 und 10 liegen.

Im folgenden wird die Erfindung anhand von Ausführungsbeispielen erläutert.

### Beispiel 1

Eine Mischung aus
12 kg Laureth-2,
10 kg Ölsäure,
7 kg Ethanoldioldistearat und
3 kg Glycerylstearat
wurde unter Rühren bei etwa 55°C zusammengeschmolzen und anschließend mit 1 kg Wasser anemulgiert, wobei mittels Ammoniak ein pH-Wert von 8,5 eingestellt wurde.

Die erhaltene stabile pumpfähige pastenförmige Voremulsion läßt sich bei Raumtemperatur leicht mit einer wäßrigen Zusammensetzung aus

| | |
|---|---|
| 2,5-Diaminotoluolsulfat | 0,75 (Gew.-%) |
| Resorcin | 0,28 |
| 3-Aminophenol | 0,03 |
| 4-Amino-3-methylphenol | 0,03 |
| 2-Amino-3-hydroxypyridin | 0,05 |
| 2-Amino-4-hydroxyethylaminoanisol | 0,03 |
| Natriumlaurylsulfat | 0,50 |
| Ammoniumchlorid | 0,25 |
| Natriumsulfit | 0,25 |
| Tetranatrium-EDTA | 0,20 |
| Ascorbinsäure | 0,15 |
| Weizenproteinhydrolysat | 0,20 |
| Parfum | 0,20 |
| Ammoniak | ad pH 10,00 |
| Wasser | ad 100,00 |

im Gewichtsverhältnis Fettphase : Wasserphase von 1 zu 2 vermischen, wobei eine stabile, wäßrige Emulsion erhalten wird.

Menschliches Haar wurde mit einem Reaktionsprodukt aus dieser Färbeemulsion und einer wäßrigen 6%-igen Wasserstoffperoxid-Zusammensetzung im Gewichtsverhältnis 1:1 25 Minuten gefärbt.
Nach dem Waschen und Trocknen wurde eine intensive lichtstabile braunviolette Färbung erhalten.

### Beispiel 2

Eine Mischung aus
10 kg Coceth-3,
10 kg Ölsäure
4 kg Ethandiol-/1,2-Propandiolstearat (1:1),
4 kg Cetylalkohol und
3,5 kg Polyglyceryl-3-methylglucosedistearat
wurde bei etwa 50°C zusammengerührt und anschließend mit 1 kg Wasser unter Zusatz von Monoethanolamin zur Einstellung eines pH-Wertes von 8,0 anemulgiert.

Die so erhaltene stabile pumpfähige pastenförmige Voremulsion läßt sich bei etwa 40°C mit einer wäßrigen Farbzusammensetzung aus

| | |
|---|---|
| 2,5-Diaminotoluolsulfat | 1,00 (Gew.-%) |
| 4-Amino-2-hydroxytoluol | 1,60 |
| 1-Naphthol | 0,40 |
| 2,5,6-Triamino-4-hydroxypyrimidinsulfat | 0,40 |
| Natriumlaurylsulfat | 1,00 |
| Natriumsulfit | 0,50 |
| Silica | 0,25 |
| Citronensäure | 0,65 |
| Panthenol | 0,25 |
| Kationisiertes Pflanzenproteinhydrolysat | 0,25 |
| Ascorbinsäure | 0,20 |
| Parfum | 0,40 |
| Monoethanolamin | 9,00 |
| Wasser | ad 100,00 |

zu einer stabilen Färbeemulsion zusammenrühren.

Im Gewichtsverhältnis 1 zu 1 mit einer 6%-igen Wasserstoffperoxid-Zusammensetzung angemischt, wird eine intensive licht- und waschstabile Violettfärbung menschlichen Haares erzielt.

## Patentansprüche

1. Voremulsion zur Herstellung eines Haarfärbemittels, umfassend eine Kombination aus
a) 20 bis 40 Gew.-% mindestens eines C₁₂-C₁₄-Fettalkoholethoxylats mit 1 bis 5 Ethylenoxidgruppen;
b) 15 bis 40 Gew.-% Ölsäure;
c) 5 bis 30 Gew.-% Ethandiol- und/oder 1,2-Propandioldistearat;
d) 5 bis 20 Gew.-% Glycerylstearat und/oder mindestens eines Zuckerfettsäureesters; und
e) 0 bis 25 Gew.-% mindestens eines C₁₂-C₁₈-Fettalkohols,
jeweils berechnet auf die Gesamtzusammensetzung der Voremulsion; enthaltend keine weiteren Emulgatoren oder Fettkörper und einen Wassergehalt von maximal 10 Gew.-%, wobei der pH-Wert auf 7,1 bis 9 eingestellt ist.

2. Voremulsion nach Anspruch 1, enthaltend als Bestandteil a) 25 bis 35 Gew.-% eines Laurylalkoholethoxylats mit 2 Ethylenoxidgruppen.

3. Voremulsion nach Anspruch 1 oder 2, enthaltend 25 bis 35 Gew.-% Ölsäure.

4. Voremulsion nach einem oder mehreren der Ansprüche 1 bis 3, enthaltend 15 bis 25 Gew.-% Ethandioldistearat.

5. Voremulsion nach einem oder mehreren der Ansprüche 1 bis 4, enthaltend als Zuckerfettsäureester 5 bis 15 Gew.-% Methylglucosesesquistearat und/oder Polyglyceryl-3-methylglucosedistearat.

6. Voremulsion nach einem oder mehreren der Ansprüche 1 bis 5, enthaltend maximal 5 Gew.-% Wasser.

7. Verwendung einer Voremulsion nach einem oder mehreren der Ansprüche 1 bis 6 zur Herstellung einer Haarfärbeemulsion.

8. Verfahren zur Herstellung einer Haarfärbeemulsion, wobei bei einer Temperatur von maximal 60°C eine Voremulsion nach mindestens einem der Ansprüche 1 bis 6 mit einer wäßrigen Zusammensetzung, die mindestens ein Oxidationshaarfarbstoff-Vorprodukt enthält, unter Rühren vermischt wird.

## Claims

1. Pre-emulsion for the preparation of a hair dyeing composition, comprising a combination of
a) 20 % to 40 % by weight of at least one C₁₂-C₁₄-fatty alcohol ethoxylate with 1 to 5 ethylene oxide groups;
b) 15 % to 40 % by weight of oleic acid;
c) 10 % to 30 % by weight of ethanediol and/or 1.2-propanediol distearate;
d) 5 % to 20 % by weight of glyceryl stearate and/or at least one sugar fatty acid ester; and,
e) 0 % to 25 % by weight of at least one C₁₂-C₁₈-fatty alcohol,
each calculated to the total pre-emulsion composition; containing no further emulsifiers or fatty substances, having a maximum water content of 10 % by weight, whereby the pH-value is between 7.1 and 9.

2. Pre-emulsion according to claim 1, comprising as component a) 25 % to 35 % by weight of a lauryl alcohol ethoxylate with 2 ethylene oxide groups.

3. Pre-emulsion according to claim 1 or 2, comprising 25 % to 35 % by weight of oleic acid.

4. Pre-emulsion according to one or more of claims 1 to 3, comprising 15 % to 25 % by weight of ethanediol stearate.

5. Pre-emulsion according to one or more of claims 1 to 4, comprising as sugar fatty acid ester 5 % to 15 % by weight of methyl glucose sesquistearate and/or polyglyceryl-3-methyl glucose distearate.

6. Pre-emulsion according to one or more of claims 1 to 5, comprising a maximum of 5 % by weight of water.

7. Use of a pre-emulsion according to one or more of claims 1 to 6 for the preparation of a hair dyeing emulsion.

8. Process for the preparation of an emulsion for the dyeing of human hair, wherein a pre-emulsion according to one of claims 1 to 6 is mixed at a maximum temperature of 60° C with an aqueous composition, comprising at least one oxidation dyestuff precursor.

## Revendications

1. Pré-émulsion pour la préparation d'une composition de coloration capillaire, comprenant une combinaison de
a) 20 à 40% en poids d'au moins un éthoxylate d'un alcool gras en C₁₂ à C₁₄ ayant 1 à 5 groupes oxyde d'éthylène;
b) 15 à 40% en poids d'acide oléique;
c) 5 à 30% en poids de distéarate d'éthanediol et/ou de distéarate de 1,2-propanediol;
d) 5 à 20% en poids de stéarate de glycéryle et/ou au moins un ester d'un acide gras saccharique; et
e) 0 à 25% en poids d'un alcool gras en C₁₂ à C₁₈, respectivement calculés par rapport à la composition totale de la pré-émulsion;
ne contenant pas d'émulsifiant ou corps gras supplémentaire et contenant une teneur en eau de 10% en poids au plus, la valeur du pH étant ajustée de 7,1 à 9.

2. Pré-émulsion selon la revendication 1, contenant en tant que composant a) 25 à 35% en poids d'un éthoxylate d'alcool laurylique avec 2 groupes oxyde d'éthylène.

3. Pré-émulsion selon la revendication 1 ou 2, contenant 25 à 35% en poids d'acide oléique.

4. Pré-émulsion selon l'une ou plusieurs des revendications 1 à 3, contenant 15 à 25% en poids de distéarate d'éthanediol.

5. Pré-émulsion selon l'une ou plusieurs des revendications 1 à 4, contenant en tant qu'ester d'acide gras saccharique, 5 à 15% en poids de sesquistéarate de méthylglucose et/ou de poly(3-méthylglucose-distéarate de glycéryle).

6. Pré-émulsion selon l'une ou plusieurs des revendications 1 à 5, contenant au plus 5% en poids d'eau.

7. Utilisation d'une pré-émulsion selon l'une ou plusieurs des revendications 1 à 6, pour la préparation d'émulsion de coloration capillaire.

8. Procédé de préparation d'une émulsion capillaire colorante, dans lequel une pré-émulsion selon l'une ou plusieurs des revendications 1 à 6, est mélangée sous agitation, à une température de 60°C au plus, avec une composition aqueuse qui contient au moins un précurseur de colorant d'oxydation capillaire.
